# EUROPEAN PATENT APPLICATION

(11) **EP 3 916 100 A1**
(43) Date of publication of application: **01.12.2021**
(21) Application number: 20176288.7
(22) Date of filing: 25.05.2020
(51) Int. Cl.: C12P 7/10, C12P 19/02, C12P 19/14, D21C 3/06

(54) **ARRANGEMENT AND METHOD FOR TREATMENT OF LIGNOCELLULOSIC BIOMASS**

(71) Applicant: Sekab E-Technology AB, 891 26 Örnsköldsvik (SE)
(72) Inventor: CAVKA, Adnan, 891 96 ARNÄSVALL (SE); SUNDVALL, Elias, 892 32 Domsjö (SE)
(74) Representative: Kransell & Wennborg KB

(57) **Abstract**

The present disclosure generally relates to a method for treatment of a lignocellulosic biomass. The method comprises the steps of pretreating the lignocellulosic biomass material in a pretreatment arrangement to form a pretreated biomass slurry; optionally hydrolysing the pretreated biomass slurry in a hydrolysis unit; retrieving gaseous SO2 from the pretreatment arrangement to provide an SO2 gas stream; treating the SO2 gas stream with an alkaline solution to provide a sulfite/bisulfite solution; supplying at least a part of the sulfite/bisulfite solution to at least one of:
a) the lignocellulosic biomass material prior to pretreating the lignocellulosic biomass material in the pretreatment arrangement;
b) the lignocellulosic biomass material in the pretreatment arrangement;
c) the pretreated biomass slurry discharged from the reactor vessel;
d) the hydrolysate or fermentable sugars of the hydrolysate.

## Description

### TECHNICAL FIELD

The present disclosure generally relates to a method for preparing an SO2 based liquid derivable from an acidified lignocellulosic biomass, to a method for preparing an SO2 gas stream derivable from an acidified lignocellulosic biomass, and to a system for treatment of lignocellulosic biomass.

### BACKGROUND

Lignocellulosic residues from forestry are attractive as feedstocks for the production of green chemicals and fuels, since they are abundant, relatively inexpensive, and not used for food. Lignocellulose consists mainly of lignin and two classes of polysaccharides, cellulose and hemicellulose. The polysaccharides can be hydrolyzed to sugars and converted to various fermentation products, e.g. bioalcohols, by means of fermenting microorganisms, such as Saccharomyces cerevisiae.

The hydrolysis of cellulose is typically preceded by a pretreatment process, in which the hemicellulose is degraded, and the cellulose is made more accessible to cellulolytic enzymes. During hydrolysis, the cellulose present is partly converted into reducing sugars.

The pretreatment process is typically carried out in a pretreatment arrangement, comprising an impregnation vessel and a reactor vessel. In the impregnation vessel, the biomass is impregnated with an additive facilitating degradation of the biomass, and in the reactor vessel the degradation is improved by exposing the biomass to an elevated temperature and pressure. The pretreatment arrangement also generally comprises an inlet for receiving the biomass to be pretreated and an outlet for discharging the pretreated biomass, typically as a biomass slurry. The overall aim of the pretreatment is to disrupt the crystalline cellulose structure and to remove or partially remove lignin from the lignocellulosic biomass. The pretreatment process is complex and involves many reactions and side-reactions. Such reactions may result in the formation of various by-products, which may be inhibitory to downstream processes. Furthermore, volatile compounds and gases contained in the lignocellulose biomass are set free when the material is degraded or partially degraded. Correspondingly, the hydrolysis is typically carried out in a hydrolysing unit and the fermentation in a fermentation unit or fermentation vessel.

The overall process, from the pre-treatment to the fermentation, is relatively complex and may comprise the addition of additives and various process streams such as water and steam. Moreover, the preferred conditions for optimizing the degradation of the biomass varies along the process. For example, in some process steps, a high amount of liquid is preferred for optimal treatment of the biomass material, while in other process steps, superfluous liquid results in unnecessary costs. Moreover, for environmental reasons, cleaning and/or removal of certain substances used, or produced, in the process are often needed. All of the above results in additional costs, and adds complexity to the process.

There is thus a need for improvements with respect to the process which allows for a more cost efficient and/or in at least some aspects less complicated system.

### SUMMARY

In view of the above, it is an object of the present invention to provide improvements with respect to methods and arrangements for the treatment of lignocellulosic biomass, particularly to achieve a less costly process, and/or in at least some aspects, a less complicated system.

According to a first aspect of the present invention, a method for preparing an SO2 based liquid derivable from an acidified lignocellulosic biomass is provided. The method comprising the steps of:
pretreating a lignocellulosic biomass material in a pretreatment arrangement, the pretreating including treating the lignocellulosic biomass material with SO2 and subjecting SO2 impregnated biomass to an elevated temperature and pressure in a reactor vessel of the pretreatment arrangement to provide a pretreated biomass slurry;
optionally hydrolyzing the pretreated biomass slurry in a hydrolysis unit with at least one aqueous hydrolyzing liquid, comprising saccharification enzymes, under conditions in which at least a part of the pretreated biomass slurry is hydrolyzed to a hydrolysate, said hydrolysate comprising fermentable sugars and inhibitory substances;
retrieving gaseous SO2 from the pretreatment arrangement to provide an SO2 gas stream;
treating the SO2 gas stream with an alkaline solution to provide a sulfite/bisulfite solution;
supplying at least a part of the sulfite/bisulfite solution to at least one of:
   a) the lignocellulosic biomass material prior to pretreating the lignocellulosic biomass material in the pretreatment arrangement;
   b) the lignocellulosic biomass material in the pretreatment arrangement, e.g. the reactor vessel;
   c) the pretreated biomass slurry discharged from the reactor vessel;
   d) the hydrolysate or fermentable sugars of the hydrolysate.

Hereby, an efficient yet simple way of re-using at least some substances from the process are provided. Besides the advantageous effects presented below for each one of the options a) - d), by re-using at least some substances a cost-effective process is provided. Moreover, by re-using at least some substances from the process, required cleaning and removal of substances e.g. due to emission restrictions, can be reduced or even omitted. In particular, the invention provides in addition to the process advantages also an efficient way of reducing SO2 (sulfur dioxide) related emissions.

The sulfite/bisulfite solution may thus be used in the process according to any one of (e.g. according to several of) options a) - d). Stated differently, the method comprises the step of supplying at least a part of the sulfite/bisulfite solution to the process upstream of, to, and/or downstream of the pretreatment of the lignocellulosic biomass material. At least a part of the sulfite/bisulfite solution may e.g. be at least 50 %, or at least 75 %, such as e.g. approximately 100 % or 100 % of the sulfite/bisulfite solution. According to at least one example embodiment, at least a part of the sulfite/bisulfite solution is less than 50 %, or less than 25 %, such as e.g. approximately 10 % of the sulfite/bisulfite solution.

According to at least one example embodiment, the step of pretreating the lignocellulosic biomass material in a pretreatment arrangement comprises treating the lignocellulosic biomass material with SO2 in an impregnation vessel of the pretreatment arrangement, and subjecting SO2 impregnated biomass from the impregnating step to the elevated temperature and pressure in the reactor vessel. According to at least one example embodiment, the step of pretreating the lignocellulosic biomass material in a pretreatment arrangement comprises providing SO2 pre-impregnated lignocellulosic biomass and subjecting the SO2 pre-impregnated lignocellulosic biomass to the elevated temperature and pressure in the reactor vessel.

In accordance with option a) the sulfite/bisulfite solution is used at least to impregnate the lignocellulosic biomass material in order to facilitate the mechanism in which the hemicellulose is degraded, and the cellulose is made more accessible to cellulolytic enzymes. Moreover, by impregnating the lignocellulosic biomass prior to the pretreatment arrangement, less SO2 may be needed in the impregnation step in the pretreatment, or the impregnation step in the pretreatment may be omitted. Thus, according to at least one example embodiment, the impregnation step using SO2 in the pretreatment arrangement may be omitted, and the sulfite/bisulfite treated lignocellulosic biomass material may be supplied directly to the reactor vessel of the pretreatment arrangement.

In accordance with option b) the sulfite/bisulfite solution may be used to impregnate the lignocellulosic biomass material for the same reasons mentioned in option a), but by supplying the sulfite/bisulfite solution to the lignocellulosic biomass material in the pretreatment arrangement, e.g. into the impregnation vessel. Here, the sulfite/bisulfite solution may be used as a complement to the SO2 supplied to the impregnation vessel. Additionality or alternatively, the sulfite/bisulfite solution can be supplied to the reactor vessel as makeup liquid (for dilution). Hereby, SO2 can be re-used in the process, as well as reducing the need of other makeup liquids (as e.g. makeup water). Moreover, this may lead to a reduced need of handling residue liquids, such as residue water, downstream of the pretreatment arrangement.

In accordance with options a) and/or b), the sulfite/bisulfite solution is typically subject to an environment with a low pH and/or high temperature, either as a direct effect of the point of application/supply (i.e. at a) or b)) or as a separate means arranged prior to the point of application/supply, which results in the formation of SO2 in gaseous form, facilitating impregnation and/or reaction with the lignocellulosic biomass material.

In accordance with option c) the sulfite/bisulfite solution is used at least to modify the characteristics of the pretreated biomass slurry prior to subjecting it to downstream process steps. Such modification is desired as the pretreated biomass slurry typically is not optimized for the downstream process steps, which is further discussed below. The supply of the sulfite/bisulfite solution according to option c) may be performed by supplying the sulfite/bisulfite solution to a process location downstream the reactor vessel, e.g. between the reactor vessel and the hydrolyzing unit.

In accordance with option d) the sulfite/bisulfite solution may be used at least to prevent undesired microbial growth of the fermentable sugars to facilitate storing of the same. For example, the fermentable sugars may initially be brought to a higher concertation than the target concentration, and subsequently be subject to dilution by the sulfite/bisulfite solution to reach the target concentration while additionally providing for the previously mentioned prevention of undesired microbial growth.

According to at least one example embodiment, the method comprises the step of detoxifying the hydrolysate by decreasing the concentration of at least one of the inhibitory substances using the sulfite/bisulfite solution.

Hereby, the resulting hydrolysate is less toxic with regards to yeast and/or other enzymes used in downstream process steps. It should be noted that the inhibitory substances are typically still present in the hydrolysate after the dilution with the sulfite/bisulfite solution, but that the inhibitory substances undergo reactions resulting in less toxic states.

According to at least one example embodiment, the step of detoxifying the hydrolysate is carried out by option c) above. However, it should be noted that any upstream supply of the sulfite/bisulfite solution at least to some extent contributes to the advantageous effects mentioned in relation to any downstream option of supply of the sulfite/bisulfite. For example, even if the sulfite/bisulfite solution is supplied in accordance with option a) or b) the advantageous effects of option c) or the detoxification of the hydrolysate, are still to at least some degree met, as the substances of the sulfite/bisulfite solution are following the flow of biomass further downstream of the point of application.

According to at least one example embodiment, the method comprises the step of increasing the pH of the pretreated biomass slurry to between 4 and 6, using the sulfite/bisulfite solution.

Hereby, a desired pH, e.g. with regards to preferred pH for enzymes, for the downstream process steps is provided. Moreover, the use of another means, such as e.g. an alkaline solution, for increasing the pH can be reduced or even omitted. However, it should be understood that the sulfite/bisulfite solution may be used together with such means (e.g. another alkaline solution) to reach the desired pH. For example, the sulfite/bisulfite solution may have a pH between 4 and 7, such as e.g. between 5 and 6, while the pretreated biomass slurry may have a pH of between 1 and 2, such as e.g. between 1.3 and 1.8. By an appropriate amount of sulfite/bisulfite solution, and possibly another alkaline solution, the target pH may be reached.

According to at least one example embodiment, the method comprises the step of increasing the pH of the pretreated biomass slurry to between 3 and 7, using the sulfite/bisulfite solution.

Hereby, the process can be adapted based on the pH with regards to preferred environment for various enzymes. For example, a relatively high pH may be desired if cellulolytic bacteria or fungi is used in downstream process steps.

It should be noted that SO2 can be retrieved from other process steps besides the pretreatment arrangement, e.g. the hydrolysis step or any fermentation step, or any auxiliary equipment arranged in between, e.g. a flash tank. The SO2 retrieved from the process is typically excess gaseous SO2.

According to at least one example embodiment, the step of treating the SO2 gas stream with an alkaline solution is performed in an SO2 scrubber.

Hereby, a well-suited equipment can be used to produce the sulfite/bisulfite solution. An SO2 scrubber is moreover relatively easy to implement in the process as it is adapted for separated streams of SO2 and alkaline solution. Moreover, the resulting sulfite/bisulfite solution is relatively easy withdrawn from the SO2 scrubber and can thus be used in the process accordingly.

According to at least one example embodiment, the method comprises the step of separation of the pretreated biomass slurry to remove a liquid fraction.

For example, the liquid fraction may be removed from the process, and the solid fraction may be used in the downstream process steps. Hereby, the process can be carried out in a more efficient manner. It should be noted that in embodiments with such separation, the supply of the sulfite/bisulfite solution according to option c) may be performed by supplying the sulfite/bisulfite solution to a process location between the reactor vessel and the separation.

According to at least one example embodiment, the method comprises a steam explosion step, preferably arranged downstream of the reactor vessel.

Hereby the biomass material is further disintegrated facilitating the degradation process.

According to at least one example embodiment, the method comprises subjecting the fermentable sugars of the hydrolysate to fermentation in an aqueous liquid utilizing at least one fermenting microorganism under conditions in which at least a part of the fermentable sugars is fermented into a primary target chemical.

Such fermentation may e.g. be carried out in a fermentation unit or fermentation vessel. The primary target chemical may e.g. be ethanol.

According to at least a second aspect of the present invention, a method for preparing a SO2 gas stream derivable from an acidified lignocellulosic biomass is provided. The method comprising the steps of:
pretreating a lignocellulosic biomass material in a pretreatment arrangement the pretreating including treating the lignocellulosic biomass material with SO2 in an impregnation vessel of the pretreatment arrangement, and subjecting SO2 impregnated biomass from the impregnating step to an elevated temperature and pressure in a reactor vessel of the pretreatment arrangement to provide a pretreated biomass slurry;
optionally hydrolyzing the pretreated biomass slurry in a hydrolysis unit with at least one aqueous hydrolyzing liquid, comprising saccharification enzymes, under conditions in which at least a part of the pretreated biomass slurry is hydrolyzed to a hydrolysate, said hydrolysate comprising fermentable sugars and inhibitory substances;
retrieving gaseous SO2 from the pretreatment arrangement to provide an SO2 gas stream;
treating the SO2 gas stream with an alkaline solution to provide a sulfite/bisulfite solution;
treating the sulfite/bisulfite solution to form a separated gas stream;
using the separated gas stream in at least one of the following processes:
   i) the impregnation vessel of the pretreatment arrangement to pretreat the lignocellulosic biomass material with SO2 present in the separated gas stream;
   ii) cooling the separated gas stream to form an SO2 liquid stream, and separating SO2 from other gaseous substance(s) present in the separated gas stream.

Effects and features of the steps of pretreating, optionally hydrolyzing, retrieving gaseous SO2 and treating the SO2 gas stream with an alkaline solution in this second aspect of the invention are largely analogous to the corresponding steps described above in connection with the first aspect of the invention. Embodiments mentioned in relation to these steps in the first aspect of the invention are largely compatible for the corresponding steps in the second aspect of the invention, of which some are exemplified below (typically without repeating the advantageous effects).

For example, the step of treating the SO2 gas stream with an alkaline solution may be performed in an SO2 scrubber, and the method may comprise the step of separation of the pretreated biomass slurry to remove a liquid fraction. Moreover, the method may comprise a steam explosion step, preferably arranged downstream of the reactor vessel. Additionality or alternatively, the method may comprise subjecting the fermentable sugars of the hydrolysate to fermentation in an aqueous liquid utilizing at least one fermenting microorganism under conditions in which at least a part of the fermentable sugars is fermented into a primary target chemical. Such fermentation may e.g. be carried out in a fermentation unit or fermentation vessel.

By treating the sulfite/bisulfite solution to form a separated gas stream and using the separated gas stream in at least one of process i) and ii), the SO2 added to the process can be re-used as a gas. Hereby, an efficient but yet simple way of re-using the SO2 from the process is provided. Besides the advantageous effects presented below for each one of the process i) and ii), by re-using at least SO2, a cost-effective process is provided. Moreover, by re-using at least SO2 from the process, required cleaning and removal of SO2 e.g. due to emission restrictions, can be reduced or even omitted.

In accordance with the process i) the gaseous SO2 can be used to impregnate the lignocellulosic biomass material in the pretreatment arrangement, and thus externally provided SO2 can be reduced or even omitted.

In accordance with the process ii) the gaseous SO2 can be cooled to form an SO2 liquid stream, which advantageously can be used for various purposes, e.g. in accordance with the first aspect of the invention, and in particular to at least one of options a) - d).

According to at least one example embodiment, the step of treating the sulfite/bisulfite solution to form a separated gas stream comprises lowering the pH of the sulfite/bisulfite solution to reduce the solubility of SO2 in the solution.

Hereby, the gaseous SO2 may more easily be separate from the solution.

The method may further comprise the step of separating at least gaseous SO2 from the sulfite/bisulfite solution to form the separated gas stream, preferably by using heat.

Hereby, a simple but yet effective means for creating the separated gas stream is provided. For example, steam may be used to heat the sulfite/bisulfite solution.

The method may further comprise the step of recirculating the SO2 liquid stream into the pretreatment, hydrolyzing and/or fermentation step.

According to at least a third aspect of the invention, a system for treatment of lignocellulosic biomass is provided. The system comprises:
- a pretreatment arrangement configured to treat the lignocellulosic biomass material with SO2, said pretreatment arrangement comprising a reactor vessel configured to subject the SO2 impregnated biomass to an elevated temperature and pressure to form a pretreated biomass slurry, the pretreatment arrangement having an upstream inlet for receiving the lignocellulosic biomass and a downstream outlet for discharging the pretreated biomass slurry;
- optionally, a hydrolysis unit arranged in fluid communication with and downstream of said pretreatment arrangement, the hydrolysis unit being configured to hydrolyze the pretreated biomass slurry with at least one aqueous hydrolyzing liquid comprising saccharification enzymes, under conditions in which at least a part of the pretreated biomass slurry is hydrolyzed to a hydrolysate, said hydrolysate comprising fermentable sugars and inhibitory substances;
- a retrieving arrangement configured to retrieve gaseous SO2 from the pretreatment arrangement and to provide an SO2 gas stream;
- an SO2 treatment unit arranged in fluid communication with the retrieving arrangement, the SO2 treatment unit being configured to treat the SO2 gas stream with an alkaline solution to provide a sulfite/bisulfite solution.

Such system may be used to carry out the method in accordance with both the first and second aspect of the invention. Thus, effects and features of this third aspect of the invention are largely analogous to those described above in connection with the first and second aspects of the invention. Embodiments mentioned in relation to the first and second aspects of the invention are largely compatible with the third aspect of the invention, of which some are exemplified below (typically without repeating the advantageous effects).

According to at least one example embodiment, the SO2 treatment unit comprises an inlet for receiving the alkaline solution, and/or an outlet for discharging the sulfite/bisulfite solution from the SO2 treatment unit.

According to at least one example embodiment, the pretreatment arrangement comprises an impregnation vessel configured to treat the lignocellulosic biomass with SO2, and a reactor vessel configured to subject the SO2 impregnated biomass from the impregnating vessel to an elevated temperature and pressure to provide said pretreated biomass slurry.

Hereby, an effective way of pretreating the lignocellulosic biomass is provided.

According to at least one example embodiment, the system further comprises a recirculation arrangement configured to supply at least a part of the sulfite/bisulfite solution to at least one of:
a) the lignocellulosic biomass material prior to the inlet of the pretreatment arrangement;
b) the lignocellulosic biomass material in the pretreatment arrangement, e.g. the reactor vessel;
c) the pretreated biomass slurry discharged from the reactor vessel (e.g. prior to subjecting the pretreated biomass slurry to at least one aqueous hydrolyzing liquid);
d) the hydrolysate or fermentable sugars of the hydrolysate.

Effects, features and embodiments of this recirculation arrangement are largely analogous to those described above in connection with the method steps according to options a) - d) in accordance with the first aspect of the invention. The recirculation arrangement may e.g. comprise transport piping, applicable valves and tanks/reservoirs and corresponding control means.

According to at least one example embodiment, the system further comprises:
- a sulfite/bisulfite treatment unit configured to treat the sulfite/bisulfite solution to form a separated gas stream;
and at least one of the following:
- a gas recirculation arrangement configured to supply the separated gas stream to the impregnation vessel of the pretreatment arrangement to pretreat the lignocellulosic biomass material with SO2 present in the separated gas stream,
- a condenser configured to cool the separated gas stream to form a SO2 liquid stream and configured to separate the SO2 from other gaseous substance(s) present in the separated gas stream.

It should be noted that the system may comprise both the gas recirculation arrangement and the condenser. Effects, features and embodiments of the gas recirculation arrangement and the condenser are largely analogous to those described above in connection with the method steps according to process i) and ii) in accordance with the second aspect of the invention. The gas recirculation arrangement may e.g. comprise transport piping, applicable valves and tanks/reservoirs and corresponding control means.

According to at least one example embodiment, the system further comprises a steam explosion configuration arranged downstream, or in an outlet region of, the reactor vessel.

According to at least one example embodiment, the system further comprises a fermentation unit arranged in fluid communication with and downstream of said hydrolysis unit, the fermentation unit being configured to subject the fermentable sugars of the hydrolysate to fermentation in an aqueous liquid utilizing at least one fermenting microorganism under conditions in which at least a part of the fermentable sugars are fermented into a primary target chemical.

The primary target chemical may e.g. be ethanol.

According to at least one example embodiment, the system comprises a control unit configured to perform at least one of the method steps of the first and/or second aspect of the invention. For example, the system may comprise additional units and components known to those skilled in the art. For example, a separation unit may be arranged between the pretreatment arrangement and the hydrolysis unit, and/or between the hydrolysis unit and the fermentation unit.

It should be understood that the wording "process" in the application text generally indicates the process from providing a lignocellulosic biomass material prior to subjecting it to the pretreatment (or alternatively from the step pretreating a lignocellulosic biomass material in a pretreatment arrangement) to discharging the pretreated biomass slurry from the reactor vessel, or discharging the hydrolysate from the hydrolysis unit or discharging the primary target chemical from the fermentation step.

### BRIEF DESCRIPTION OF THE DRAWINGS

The various aspects of the present invention, including its particular features and advantages, will be readily understood from the following detailed description and the accompanying drawings, in which:
Fig. 1 illustrates a system according to an exemplary embodiment of the present invention;
Fig. 2 schematically illustrates the steps of a method for preparing an SO2 based liquid according to an exemplary embodiment of the present invention, and
Fig. 3 schematically illustrates the steps of a method for preparing an SO2 gas stream according to an exemplary embodiment of the present invention.

### DETAILED DESCRIPTION

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

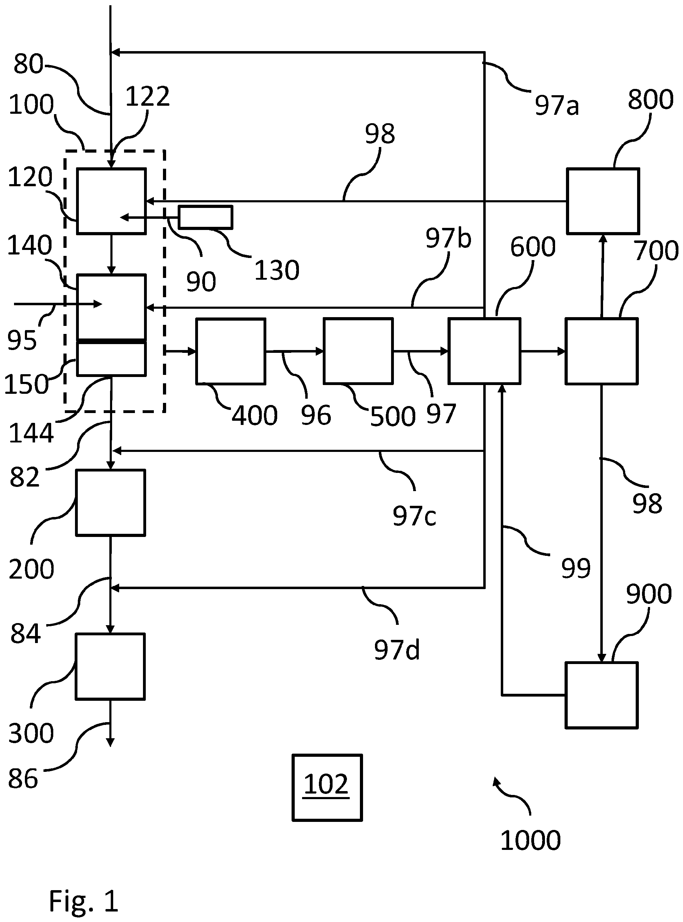

Fig. 1 illustrates a system 1000 for treatment of lignocellulosic biomass. The system comprises a pretreatment arrangement 100, a hydrolysis unit 200 and a fermentation unit 300, which all will be described in more detail in the following.

In the embodiment of Fig. 1, the pretreatment arrangement 100 comprises an impregnation vessel 120 configured to treat the lignocellulosic biomass with SO2, and a reactor vessel 140 arranged downstream of the impregnation vessel 120. The reactor vessel 140 is configured to subject the SO2 impregnated biomass from the impregnating vessel 120 to an elevated temperature and pressure to provide a pretreated biomass slurry 82. The pretreatment arrangement 100 comprises an upstream inlet 122 for receiving the lignocellulosic biomass material 80, and a downstream outlet 144 for discharging the biomass as a pretreated biomass slurry 82. Moreover, in an outlet portion of the reactor vessel 140, a steam explosion configuration 150 is arranged.

The lignocellulosic biomass may be, but is not limited to, hardwoods, softwoods, sugarcane bagasse, energy cane, corn stover, corn cobs, corn fibers, straw from rice, wheat, rye and other crop or forestry residues. As illustrated by the arrow 95, steam and/or additional catalysts may in embodiments be added to the reactor vessel 140 for certain pretreatment conditions.

The pretreatment arrangement 100 further comprises an SO2 injection arrangement 130 configured to supply an SO2 feed 90 to the lignocellulosic biomass in the impregnation vessel 120. The SO2 injection arrangement 130 may include a controllable valve (not shown) having an adjustable opening configuration for varying the SO2 feed. Typically, the SO2 injection arrangement 130 is configured to supply the SO2 to the impregnation vessel 120 in gaseous form, but a configuration in which the SO2 is fed to the impregnation vessel 120 as a liquid is within the scope of the invention.

In short, the pretreatment arrangement 100 operates as follows. The lignocellulosic biomass material 80 enters the pretreatment arrangement 100, and the impregnation vessel 120, via the upstream inlet 122. In the impregnation vessel 120, the lignocellulosic biomass is treated or impregnated with SO2 by the use of the SO2 injection arrangement 130. The operating temperature of the impregnation vessel 120 may e.g. be in the range of 20 °C to 90 °C, e.g. between 20 °C to 60 °C. Subsequently, the SO2 impregnated biomass from the impregnation vessel 120 is fed to the reactor vessel 140. In the reactor vessel 140, and during operation, the SO2 impregnated biomass from the impregnation vessel 120 is subject to an elevated temperature and pressure forming a biomass slurry. For example, the temperature in the reactor vessel 140 during pretreatment may be in the interval of 185 °C to 225 °C, such as 200 °C to 215 °C, and the pressure in the interval of 10 to 25 bar, such as 15 to 20 bar. The reactor vessel 140 may according to one example embodiment be a vertical reactor vessel. However, horizontal, as well as inclined, reactor vessels are also conceivable for the purpose of the present disclosure. The pretreatment arrangement 100 may further comprise specific types of inlets (such as e.g. an inlet hopper), feeding means (e.g. a plug screw feeder) for transporting the biomass material internally of the pretreatment arrangement 100, and moisture adjusting units (e.g. compressing, dewatering units) known to those skilled in the art.

The hydrolysis unit 200 is arranged in fluid communication with, and downstream of, the pretreatment arrangement 100. The hydrolysis unit 200 is configured to hydrolyze the pretreated biomass slurry 82 with at least one aqueous hydrolyzing liquid comprising saccharification enzymes, under conditions in which at least a part of the pretreated biomass slurry is hydrolyzed to a hydrolysate 84. The hydrolysate 84 comprises fermentable sugars and inhibitory substances.

The fermentation unit 300 is arranged in fluid communication with, and downstream of, the hydrolysis unit 200. The fermentation unit is configured to subject the fermentable sugars of the hydrolysate to fermentation in an aqueous liquid utilizing at least one fermenting microorganism under conditions in which at least a part of the fermentable sugars is fermented into a primary target chemical 86. The primary target chemical can e.g. be ethanol.

The system 1000 in Fig. 1 comprises a retrieving arrangement 400. The retrieving arrangement 400 is configured to retrieve gaseous SO2 from at least the pretreatment arrangement 100, and to provide an SO2 gas stream 96. As can be seen in Fig. 1, the system 1000 further comprises a SO2 treatment unit 500 arranged in fluid communication with the retrieving arrangement 400, and thus configured to receive the SO2 gas stream 96 from the retrieving arrangement 400. The SO2 treatment unit 500 in Fig. 1 is a SO2 scrubber 500, configured to treat the SO2 gas stream 96 with an alkaline solution to form a sulfite/bisulfite solution 97.

The system 1000 in Fig. 1 comprises a recirculation arrangement 600 configured to supply at least a part of the sulfite/bisulfite solution 97 to at least one of the pretreatment arrangement 100, the hydrolyzing unit 200, the fermentation unit 300, and any intermediate component/equipment fluidly connecting the arrangement and/or units. As can be seen in Fig. 1, several possibilities for supplying the sulfite/bisulfite solution 97 to the process is presented. According to a first option a) the sulfite/bisulfite solution h is transferred to the lignocellulosic biomass material 80 prior to the inlet 122 of the pretreatment arrangement 100. Hereby, the sulfite/bisulfite solution 97a may be used to impregnate the lignocellulosic biomass material in order to facilitate the mechanism in which the hemicellulose is degraded, and the cellulose is made more accessible to cellulolytic enzymes. According to a second option b) the sulfite/bisulfite solution 97b is transferred to the lignocellulosic biomass material (e.g. SO2 impregnated biomass), or biomass slurry in the pretreatment arrangement 100, here exemplified as to the reactor vessel 140. Hereby, the sulfite/bisulfite solution 97b may be used to impregnate the lignocellulosic biomass with the same result in option a) or used as makeup liquid in the reactor vessel 140. According to a third option c) the sulfite/bisulfite solution 97c is transferred to the pretreated biomass slurry 82 discharged from the reactor vessel 140, at least prior to subjecting the pretreated biomass slurry to at least one aqueous hydrolyzing liquid (as e.g. in the hydrolyzing unit 200). Hereby, the sulfite/bisulfite solution 97c is used at least to modify the characteristics of the pretreated biomass slurry prior to subjecting it to downstream process steps, e.g. by detoxifying the hydrolysate by decreasing the concentration of at least one of the inhibitory substances and/or increasing the pH of the pretreated biomass slurry. According to a fourth option d) the sulfite/bisulfite solution 97d is transferred to the hydrolysate or fermentable sugars of the hydrolysate. Hereby, the sulfite/bisulfite solution may be used to prevent undesired microbial growth of the fermentable sugars to facilitate storing of the same.

In addition to, or as an alternative to, the recirculation arrangement 600, the system 100 may comprise a sulfite/bisulfite treatment unit 700 configured to treat the sulfite/bisulfite solution to form a separated gas stream 98. The sulfite/bisulfite treatment unit 700 may e.g. comprise a flash tank, or other separating unit, in which heat (e.g. steam) is used to cause a separation of gaseous substances from the sulfite/bisulfite solution 97. For example, the amount of SO2 in the separated gas stream 98 can be increased by reducing the pH of the sulfite/bisulfite solution 97 prior to, or in, the sulfite/bisulfite treatment unit 700. Hereby, the solubility of SO2 in the sulfite/bisulfite solution 97 is reduced, thereby facilitating gaseous removal of the same.

The system 1000 may further comprise a gas recirculation arrangement 800 arranged downstream of the sulfite/bisulfite treatment unit 700. The gas recirculation arrangement 800 is configured to supply the separated gas stream 98 back to the pretreatment arrangement 100, and here specifically to the impregnation vessel 120. Hereby, the SO2 in the separated gas stream 98 can be used to pretreat the lignocellulosic biomass material. Additionality, or alternatively, the separated gas stream 98 can be directed to a condenser 900 configured to cool the separated gas stream 98 to form an SO2 liquid stream 99. Hereby, the SO2 will furthermore be separated from other gaseous substance(s) present in the separated gas stream 98. The SO2 liquid stream 99 can e.g. be recirculated into the system 1000 via the recirculation arrangement 600 according to at least one of options a) - d) previously described.

The system 1000 may also comprise a product recovery unit, such as distillation or ion exchange chromatography, arranged downstream of and in fluid communication with the fermentation unit 300.

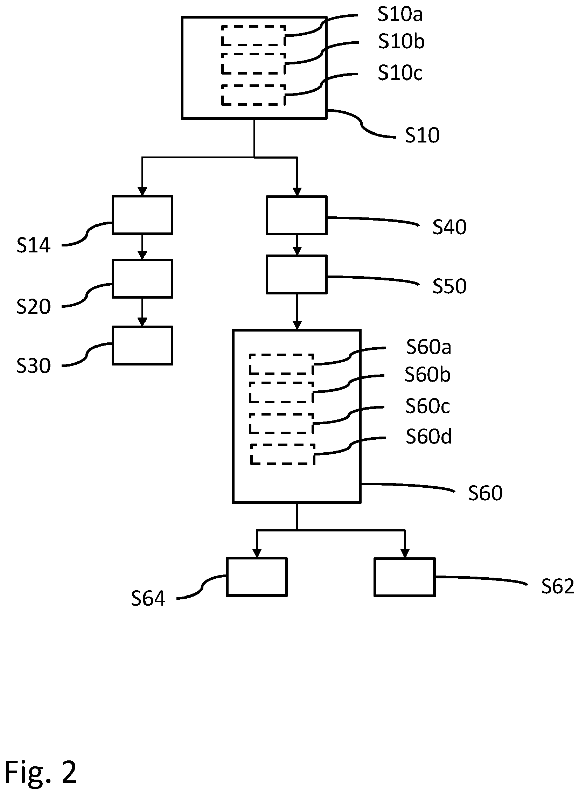

An embodiment of the invention will now be described with reference to the flow chart presented in Fig. 2. Fig. 2 schematically illustrates the steps of a method for preparing an SO2 based liquid derivable from an acidified lignocellulosic biomass according to at least one example embodiment of the invention. The arrangements and units described with reference to Fig. 2 may e.g. be those described with reference to Fig. 1.

First, the main steps, and significant optional steps of the method are described, where after further optional steps are presented. In step S10, a lignocellulosic biomass material is pretreated in a pretreatment arrangement (e.g. pretreatment arrangement 100 in Fig. 1). The pretreating may include the sub-step S10A of impregnating the lignocellulosic biomass with an SO2 feed in an impregnation vessel (e.g. impregnation vessel 120 in Fig. 1), and sub-step S10B of subjecting SO2 impregnated biomass from the impregnating step S10A to an elevated temperature and pressure in a reactor vessel (e.g. reactor vessel 140 in Fig. 1). After the pretreatment arrangement, a pretreated biomass slurry is provided.

In an optional step S20, the pretreated biomass slurry is hydrolysed in a hydrolysis unit (e.g. the hydrolysis unit 200 in Fig. 1) with at least one aqueous hydrolysing liquid. The hydrolysing liquid typically comprises saccharification enzymes, wherein at least a part of the pretreated biomass slurry is hydrolysed to a hydrolysate comprising fermentable sugars and inhibitory substances.

In an optional step S30 the fermentable sugars of the hydrolysate is subject to fermentation in an aqueous liquid utilizing at least one fermenting microorganism under conditions in which at least a part of the fermentable sugars are fermented into a primary target chemical, e.g. ethanol (e.g. by utilizing the fermentation unit 300 in Fig. 1). In other words, fermentation of the hydrolysate into a target chemical is performed by means of fermenting organism, such as bacteria and/or yeast in the fermentation unit.

It should be noted that step S20 and step S30 are optional and that the following steps of the method can be carried out based on the pretreated biomass slurry from the pretreatment arrangement.

In step S40, gaseous SO2 is retrieved from the pretreatment arrangement to provide an SO2 gas stream (e.g. by utilizing the retrieving arrangement 400 in Fig. 1). For example, excess SO2 can be removed from the reactor vessel of the pretreatment arrangement, e.g. by utilizing a gas valve fluidly connected to the reactor vessel. Excess SO2 may alternatively, or additionality, be removed from the impregnation vessel of the pretreatment arrangement or the hydrolysing unit or fermentation unit.

In a step S50, the SO2 gas stream from step S40 is treated with an alkaline solution to provide a sulfite/bisulfite solution (e.g. by utilizing the SO2 treatment unit 500 in Fig. 1). For example, an SO2 scrubber may be used for step S50. The alkaline solution may e.g. be sprayed into a gas cushion of the SO2 gas in order to increase the contact area between the SO2 gas and the alkaline solution.

In step S60, at least a part of the sulfite/bisulfite solution from step S50 is supplied to the process (e.g. by utilizing the recirculation arrangement 600 in Fig. 1), and at least a part of content in the sulfite/bisulfite solution is thus re-used in the process (recirculated). The sulfite/bisulfite solution may be supplied to the process according to at least one of the following sub-steps:

In sub-step S60a, the sulfite/bisulfite solution is supplied to the lignocellulosic biomass material prior to pretreating the lignocellulosic biomass material in the pretreatment arrangement, i.e. prior to step S10. As mentioned in relation to Fig. 1, this is beneficial at least because the sulfite/bisulfite solution may be used to impregnate the lignocellulosic biomass material in order to facilitate the mechanism in which the hemicellulose is degraded, and the cellulose is made more accessible to cellulolytic enzymes.

In sub-step S60b, the sulfite/bisulfite solution is supplied to the lignocellulosic biomass material in the pretreatment arrangement, e.g. supplied to the reactor vessel. Thus, the sulfite/bisulfite solution may be used during the pretreatment step S10. As mentioned in relation to Fig. 1, this is beneficial at least because he sulfite/bisulfite solution may be used to impregnate the lignocellulosic biomass with the same result in step S60a or used as makeup liquid in the reactor vessel.

In sub-step S60c, the sulfite/bisulfite solution is supplied to the pretreated biomass slurry discharged from the reactor vessel, i.e. subsequent to step S10 but prior step S20 (i.e. prior to subjecting the pretreated biomass slurry to at least one aqueous hydrolysing liquid). That is, the sub-step S60c may be performed by supplying the sulfite/bisulfite solution to a process location downstream the reactor vessel, e.g. between the reactor vessel and the hydrolysing unit. As mentioned in relation to Fig. 1, this is beneficial at least because, the sulfite/bisulfite solution is used at least to modify the characteristics of the pretreated biomass slurry prior to subjecting it to downstream process steps, e.g. by detoxifying the hydrolysate by decreasing the concentration of at least one of the inhibitory substances and/or increasing the pH of the pretreated biomass slurry.

In sub-step S60d, the sulfite/bisulfite solution is supplied to the fermentable sugars of the hydrolysate, i.e. subsequent to step S20, and possibly prior to, or during, step S30 (i.e. either prior to, or during the fermentation step). As mentioned in relation to Fig. 1, this is beneficial at least because the sulfite/bisulfite solution may be used to prevent undesired microbial growth of the fermentable sugars to facilitate storing of the same.

It should be understood that at least one of the sub-steps S60a-S60d are used in the method to supply the sulfite/bisulfite solution to the process. However, use of more than one of the sub-steps S60a-S60d, and/or other options of supplying the sulfite/bisulfite solution to the process, are within the scope of the invention.

In the following, further optional steps of the method are described, although they are not explicitly stated as optional.

In a step S10c, the biomass slurry is subject to a steam explosion, preferably arranged downstream, or in an end section of, the pretreatment arrangement or reactor vessel.

In a step S14 the pretreated biomass slurry is subject to a separation in order to remove a liquid fraction. For example, the liquid fraction is removed from the process, and the solid fraction are further used in the downstream process (as step S20 and step S30).

In a step S62 the hydrolysate is detoxified by decreasing the concentration of at least one of the inhibitory substances using the sulfite/bisulfite solution. The inhibitory substances are typically still present in the hydrolysate, but may undergo reactions resulting in less toxic states with regards to yeast and/or other enzymes used in downstream process steps. The step S62 may e.g. be embodied by sub-step S60c.

However, it should be noted that any upstream supply of the sulfite/bisulfite solution at least to some extent contributes to the advantageous effects of any downstream option of supply of the sulfite/bisulfite. For example, even if solely the sub-step S60a is utilized, the advantageous effects of sub-steps S60b, S60c and S60d, or step S62 of detoxification of the hydrolysate, are still to at least some degree met, as the substances of the sulfite/bisulfite solution are following the flow of biomass further downstream.

In a step S64, the pH of the pretreated biomass slurry is increase to between 4 and 6, or between 4.5 and 5.5, using the sulfite/bisulfite solution. The step S54 may e.g. be embodied by sub-step S60c, but as previously mentioned, also sub-steps S60a and/or sub-step S60b could be used to increase the pH of the pretreated biomass slurry as the substances causing the pH increase are following the flow of biomass further downstream.

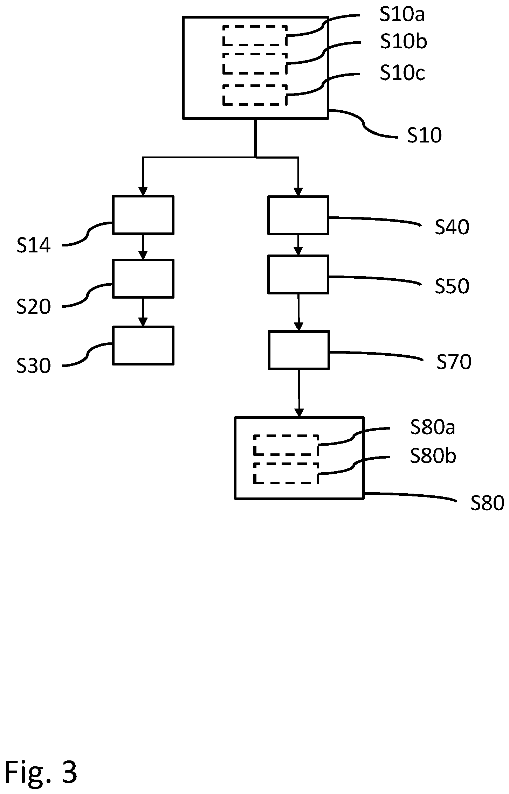

An alternative embodiment of the invention will now be described with reference to the flow chart presented in Fig. 3. Fig. 3 schematically illustrates the steps of a method for preparing an SO2 gas stream derivable from an acidified lignocellulosic biomass according to at least one example embodiment of the invention. The arrangements and units described with reference to Fig. 3 may e.g. be those described with reference to Fig. 1.

Many of the steps in Fig. 3 are equivalent to corresponding steps in Fig. 2, and are thus not explained in detail again. Particularly, steps S10 (with sub-steps S10A, S10B and/or S10C), S14, S20, S30, S40 and S50 described with reference to Fig. 2 may be identically used in the method of Fig. 3.

In a step S70 subsequent to step S50 of treating the SO2 gas stream from step S40 with an alkaline solution to provide a sulfite/bisulfite solution (e.g. by utilizing the SO2 treatment unit 500 in Fig. 1, which may be an SO2 scrubber), the sulfite/bisulfite solution is treated to form a separated gas stream (e.g. by utilizing the sulfite/bisulfite treatment unit 700). For example, the treatment in step S70 may comprise lowering the pH of the sulfite/bisulfite solution to reduce the solubility of SO2 in the solution. Additionality or alternatively, step S70 may comprise separating at least gaseous SO2 from the sulfite/bisulfite solution to form the separated gas stream by using heat (e.g. steam).

In a step S80 subsequent to step S70, the separated gas stream is utilized in the process. The separated gas stream may be used in a process according at least one of the following sub-steps:
In sub-step S80a, the separated gas stream is used to pretreat the lignocellulosic biomass material with SO2 present in the separated gas stream, e.g. in the impregnation vessel of the pretreatment arrangement. That is, SO2 earlier supplied to the process is re-circulated to again pretreat the lignocellulosic biomass material (e.g. by utilizing the recirculation arrangement 800 in Fig. 1).
In a sub-step S80b, the separated gas stream is cooled (e.g. in the condenser 900 of Fig. 1) to form an SO2 liquid stream. The sub-step S80b may comprise separating SO2 from other gaseous substance(s) present in the separated gas stream. The SO2 liquid stream may then be supplied to the process again, e.g. into the pretreatment, hydrolyzing and/or fermentation step, e.g. according to step S60 of Fig. 2.

The methods presented in Fig. 2 and Fig. 3 may comprise further optional steps, such as e.g. moisture adjusting steps (e.g. compressing or dewatering steps), and various monitoring/measuring steps. Moreover, any liquid withdrawn from such moisture adjusting step may be recirculated into the process again, potentially treated in the sulfite/bisulfite treatment unit to form a separated gas stream including SO2, and/or be provided back into the system in the same way as the sulfite/bisulfite solution (any of options a)-d)). Each method may preferably be automated and adapted for continuous operation. It should be noted that the steps according to the methods of Fig. 2 and Fig. 3, respectively, need not to be carried out in the order presented here.

Turning back to Fig. 1, system 1000 may further comprises a control unit 102 configured to perform, or send operational instructions corresponding to, one or more of the method steps described with reference to Fig 2 and/or Fig. 3.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method for preparing an SO2 based liquid derivable from an acidified lignocellulosic biomass, comprising the steps of:
pretreating a lignocellulosic biomass material in a pretreatment arrangement, the pretreating including treating the lignocellulosic biomass material with SO2 and subjecting SO2 impregnated biomass to an elevated temperature and pressure in a reactor vessel of the pretreatment arrangement to provide a pretreated biomass slurry;
optionally hydrolysing the pretreated biomass slurry in a hydrolysis unit with at least one aqueous hydrolysing liquid, comprising saccharification enzymes, under conditions in which at least a part of the pretreated biomass slurry is hydrolysed to a hydrolysate, said hydrolysate comprising fermentable sugars and inhibitory substances;
retrieving gaseous SO2 from the pretreatment arrangement to provide an SO2 gas stream;
treating the SO2 gas stream with an alkaline solution to provide a sulfite/bisulfite solution;
supplying at least a part of the sulfite/bisulfite solution to at least one of:
a) the lignocellulosic biomass material prior to pretreating the lignocellulosic biomass material in the pretreatment arrangement;
b) the lignocellulosic biomass material in the pretreatment arrangement, e.g. the reactor vessel;
c) the pretreated biomass slurry discharged from the reactor vessel;
d) the hydrolysate or fermentable sugars of the hydrolysate.

2. The method according to claim 1, wherein the step of treating the SO2 gas stream with an alkaline solution is performed in an SO2 scrubber.

3. The method according to any one of claims 1-2, further comprising the step of detoxifying the hydrolysate by decreasing the concentration of at least one of the inhibitory substances using the sulfite/bisulfite solution.

4. The method according to any one of the preceding claims, further comprising the step of increasing the pH of the pretreated biomass slurry to between 4 and 6, using the sulfite/bisulfite solution.

5. The method according to any one of the preceding claims, further comprising the step of separation of the pretreated biomass slurry to remove a liquid fraction.

6. The method according to any one of the preceding claims, wherein the supply of the sulfite/bisulfite solution according to c) is performed by supplying the sulfite/bisulfite solution to a process location downstream the reactor vessel, e.g. between the reactor vessel and the hydrolyzing unit.

7. The method according to any one of the preceding claims, further comprising a steam explosion step, preferably arranged downstream of the reactor vessel.

8. The method according to any one of the preceding claims, further comprising subjecting the fermentable sugars of the hydrolysate to fermentation in an aqueous liquid utilizing at least one fermenting microorganism under conditions in which at least a part of the fermentable sugars is fermented into a primary target chemical.

9. The method according to any one of the preceding claims, wherein the step of pretreating the lignocellulosic biomass material in a pretreatment arrangement comprises treating the lignocellulosic biomass material with SO2 in an impregnation vessel of the pretreatment arrangement, and subjecting SO2 impregnated biomass from the impregnating step to the elevated temperature and pressure in the reactor vessel.

10. A method for preparing an SO2 gas stream derivable from an acidified lignocellulosic biomass, comprising the steps of:
pretreating a lignocellulosic biomass material in a pretreatment arrangement the pretreating including treating the lignocellulosic biomass material with SO2 in an impregnation vessel of the pretreatment arrangement, and subjecting SO2 impregnated biomass from the impregnating step to an elevated temperature and pressure in a reactor vessel of the pretreatment arrangement to provide a pretreated biomass slurry;
optionally hydrolysing the pretreated biomass slurry in a hydrolysis unit with at least one aqueous hydrolysing liquid, comprising saccharification enzymes, under conditions in which at least a part of the pretreated biomass slurry is hydrolysed to a hydrolysate, said hydrolysate comprising fermentable sugars and inhibitory substances;
retrieving gaseous SO2 from the pretreatment arrangement to provide an SO2 gas stream;
treating the SO2 gas stream with an alkaline solution to provide a sulfite/bisulfite solution;
treating the sulfite/bisulfite solution to form a separated gas stream;
using the separated gas stream in at least one of the following processes:
i) the impregnation vessel of the pretreatment arrangement to pretreat the lignocellulosic biomass material with SO2 present in the separated gas stream;
ii) cooling the separated gas stream to form an SO2 liquid stream, and separating SO2 from other gaseous substance(s) present in the separated gas stream;

11. The method according to claim 10, wherein the step of treating the sulfite/bisulfite solution to form a separated gas stream comprises lowering the pH of the sulfite/bisulfite solution to reduce the solubility of SO2 in the solution.

12. The method according to any one of claims 10-11, further comprising the step of separating at least gaseous SO2 from the sulfite/bisulfite solution to form the separated gas stream, preferably by using heat.

13. The method according to any one of claims 10-12, further comprising the step of recirculating the SO2 liquid stream into the pretreatment, hydrolysing and/or fermentation step.

14. A system for treatment of lignocellulosic biomass comprising:
- a pretreatment arrangement configured to treat the lignocellulosic biomass material with SO2, said pretreatment arrangement comprising a reactor vessel configured to subject the SO2 impregnated biomass to an elevated temperature and pressure to form a pretreated biomass slurry, the pretreatment arrangement having an upstream inlet for receiving the lignocellulosic biomass and a downstream outlet for discharging the pretreated biomass slurry;
- optionally, a hydrolysis unit arranged in fluid communication with and downstream of said pretreatment arrangement, the hydrolysis unit being configured to hydrolyse the pretreated biomass slurry with at least one aqueous hydrolysing liquid comprising saccharification enzymes, under conditions in which at least a part of the pretreated biomass slurry is hydrolysed to a hydrolysate, said hydrolysate comprising fermentable sugars and inhibitory substances;
- a retrieving arrangement configured to retrieve gaseous SO2 from the pretreatment arrangement and to provide an SO2 gas stream;
- an SO2 treatment unit arranged in fluid communication with the retrieving arrangement, the SO2 treatment unit being configured to treat the SO2 gas stream with an alkaline solution to provide a sulfite/bisulfite solution.

15. The system according to claim, 14, further comprising a recirculation arrangement configured to supply at least a part of the sulfite/bisulfite solution to at least one of:
a) the lignocellulosic biomass material prior to the inlet of the pretreatment arrangement;
b) the lignocellulosic biomass material in the pretreatment arrangement, e.g. the reactor vessel;
c) the pretreated biomass slurry discharged from the reactor vessel
d) the hydrolysate or fermentable sugars of the hydrolysate.

16. The system according to any one of claims 14-15, wherein the pretreatment arrangement comprises an impregnation vessel configured to treat the lignocellulosic biomass with SO2, and wherein the reactor vessel is configured to subject the SO2 impregnated biomass from the impregnating vessel to the elevated temperature and pressure to provide said pretreated biomass slurry.

17. The system according to claim 16, further comprising
- a sulfite/bisulfite treatment unit configured to treat the sulfite/bisulfite solution to form a separated gas stream;
and at least one of the following:
- a gas recirculation arrangement configured to supply the separated gas stream to the impregnation vessel of the pretreatment arrangement to pretreat the lignocellulosic biomass material with SO2 present in the separated gas stream,
- a condenser configured to cool the separated gas stream to form a SO2 liquid stream and configured to separate the SO2 from other gaseous substance(s) present in the separated gas stream.

18. The system according to any one of claims 14-17, further comprising a steam explosion configuration arranged downstream, or in an outlet region of, the reactor vessel.

19. The system according to any one of claims 14-18, further comprising a fermentation unit arranged in fluid communication with and downstream of said hydrolysis unit, the fermentation unit being configured to subject the fermentable sugars of the hydrolysate to fermentation in an aqueous liquid utilizing at least one fermenting microorganism under conditions in which at least a part of the fermentable sugars are fermented into a primary target chemical.
